Europäisches Patentamt

European Patent Office

Office européen des brevets

(19)

(11) Publication number: **0 073 558**
A2

(12) **EUROPEAN PATENT APPLICATION**

(21) Application number: **82303162.0**

(22) Date of filing: **17.06.82**

(51) Int. Cl.³: **A 61 B 5/00**

(30) Priority: **25.08.81 US 296239**

(43) Date of publication of application: **09.03.83**
**Bulletin 83/10**

(84) Designated Contracting States: **DE FR GB NL**

(71) Applicant: **THE UNITED STATES OF AMERICA as represented by the Secretary U.S. Department of Commerce, National Technical Information Service Office of Government Inventions and Patents 5285 Port Royal Road, Springfield, Virginia 22161 (US)**

(72) Inventor: **Goldstein, Seth Richard, 5906 Kirby Road, Bethesda Maryland 20817 (US)**
Inventor: **Chen, Victor T., 16629 Cutlass Drive, Rockville Maryland 20853 (US)**
Inventor: **Markle, David Reed, 5930 North 9th. Street, Arlington Virginia 22205 (US)**

(74) Representative: **De Minvielle-Devaux, Ian Benedict Peter et al, CARPMAELS & RANSFORD 43, Bloomsbury Square, London WC1A 2RA (GB)**

(54) **Fiber optic pH probe for tissue measurements.**

(57) A fiber optic probe consisting of input and output optic fibers encased inside a small dialysis hollow tube rigidly secured inside a stainless steel needle. The dialysis tube has a reflective sealing plug element, with a dye column adjacent thereto, located between the ends of the optic fibers and the reflective plug element. The needle has two opposite slots adjacent the dye column, and the needle walls between the slots are spread outwardly or partly drilled away to provide complete exposure of the surface of the dialysis tube in this region to the surrounding tissue when the probe is inserted therein.

# FIBER OPTIC pH PROBE FOR TISSUE MEASUREMENTS

## FIELD OF THE INVENTION

This invention relates to medical probe devices, and more particularly to an improved fiber optic pH probe which is adapted to be implanted in the body of a human or of an animal for physiologocal studies.

## BACKGROUND OF THE INVENTION

In the previously used fiber optic pH probes considerable inconvenience and trauma are involved during insertion of the probe into the tissue of the patient or animal subject. For example, in a previous fiber optic pH probe described in U.S. Patent 4,200,110 to J.I. Peterson and S.R. Goldstein, the probe has an outer sheath of dialysis tubing which is about 0.3 mm in diameter and is not rigid enough to be inserted into tissue by itself. This necessitates putting it inside a large guide needle which is pushed into the tissue and then is retracted to allow the probe to be exposed. This procedure often results in damage to the probe, with resultant signal artifacts, as well as trauma to the tissue of interest, e.g., a beating heart. Also, these prior probes give relatively weak signals. There is a definite need to provide a more convenient and less traumatic means for the insertion of a probe into the tissue, and to provide an increased signal from the probe.

## SUMMARY OF THE INVENTION

In accordance with the present invention,
the probe is miniaturized, it is incorporated into a
small rigid needle, and it is provided with a
reflection surface at the end of its forward light
path to increase the amount of light entering the
return light fiber.  In the present invention, the
probe is made, for example, with very small plastic
optic fibers (of the order of 0.075 mm in diameter)
which are encased inside a small dialysis hollow fiber
(about 0.2 mm in diameter).  This assembly is rigidly
attached inside a small (approximately 0.25 mm inner
diameter and approximately 0.45 mm outer diameter)
stainless steel needle.  The needle is fabricated to
allow hydrogen ions to gain access to the dialysis
tubing substantially over the entire tubing area.
This is important to reduce the response time of the
probe, and is accomplished, for example by cutting two
opposing slots in the needle and either spreading the
remaining intervening needle tubing wall or removing
part of the inside surface of the remaining needle
wall with a drill oriented perpendicular to the
needle axis and positioned in the center of the slot.

A dye capsule employed in the probe and the
light path fibers are protected from physical trauma
since they are mainly inside the hollow needle, and
the needle is easily insertable into tissue.  Tissue
damage is minimized by this arrangement, since the
insertion and retraction of a large guide needle is
not required.  A reflecting surface, e.g., a glass
rod (which may be approximately 0.5 mm in length)
with a vapor-deposited reflective metallic coating,
is located at the end of the dye column and serves as

0073558

a mirror to enhance reflection and increase the
transmission of light through the dye path, thus
increasing the strength of the signal. It has been
demonstrated that this allows for the use of a
shorter-length dye column, which shortenes the probe
and provides greater localization of the measurement.
The reflecting element also eliminates optical
artifacts which, in the previously employed probes,
could result from different optical conditions at the
end of the probe, avoidable only by the use of a
relatively long dye column.

Accordingly, an object of the invention is to
provide for improved physiological study.

Another object is to provide an improved
fiber optic pH probe which overcomes the deficiencies
and disadvantages of the previously known fiber optic
probes.

A further object of the invention is to
provide an improved miniaturized fiber optic pH probe
which is provided with a reflective surface arranged
to increase its signal, which is of reduced length
and size, which provides a highly localized measurement
area, which is incorporated into a rigid needle, and
which is readily accessible by hydrogen ions because
of the provision of access openings or slots in the
needle.

A still further object of the invention is
to provide an improved fiber pH probe which can be
easily inserted into tissue without requiring the
use of a large guide needle, which causes minimum
damage or trauma to the tissue of interest, which
generates minimal signal artifact, and which
provides improved output signal strength.

## BRIEF DESCRIPTION OF THE DRAWINGS

Further objects and advantages of the invention will become apparent from the following description and claims, and from the accompanying drawings, wherein:

Fig. 1 is a perspective view of the separated main components of an improved fiber optic pH probe according to the present invention, namely, the forward portion of the hollow needle and the package of parts adapted to be housed therein.

Fig. 2 is an enlarged longitudinal vertical cross-sectional view taken through an assembled probe unit comprising the components shown in Fig. 1.

Fig. 3 is an enlarged top plan view taken substantially on the line 3-3 of Fig. 2.

Fig. 4 is an enlarged plan view similar to Fig. 3 which shows a variation of the technique for separating the membrane from the needle.

## DETAILED DESCRIPTION OF A PREFERRED EMBODIMENT

Referring to the drawings, an improved fiber optic pH probe 11 constructed in accordance with the present invention comprises a small hollow rigid stainless steel needle 12 having the cyclindrical inner lumen 13 and having a bevelled tissue-penetrating pointed forward end 14. In a typical embodiment, the needle had an outer diameter of 0.45 mm and an inner diameter of 0.25 mm. The needle is formed near its forward end with relatively large opposing slots 15, 15 with outwardly spread intervening wall portions 16, 16, as shown in Fig. 3, or drilled as shown in Fig. 4 with reduced wall portions 23, 23.

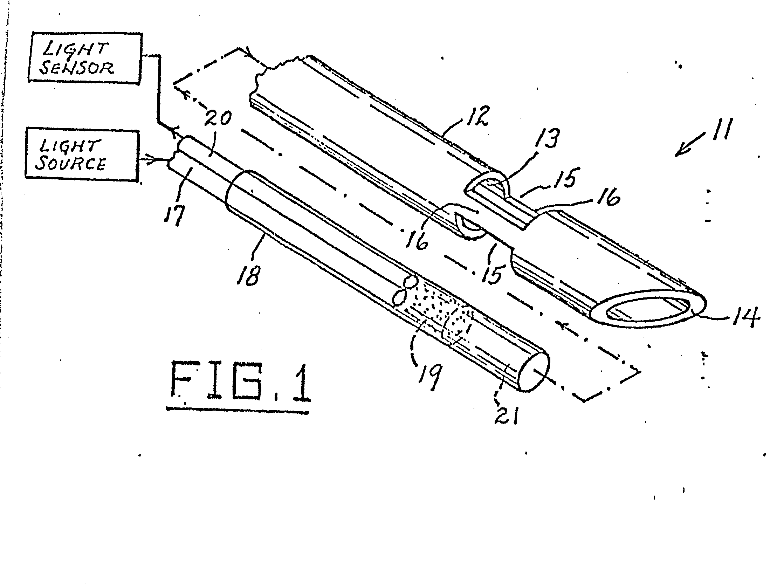

As shown in Fig. 1, the probe 11 is provided with
a pair of very small optic fibers 17, 20, of the order
of 0.075 mm in diameter, which are encased inside a
small dialysis fiber 18, approximately 0.2 mm in diameter. A small dye column 19 is contained in the dialysis
tubing 18 forwardly adjacent to the ends of the optic
fibers 17, 20, for indicating pH. The dye-containing
material 19 may be any of such known materials, which,
when employed in conjunction with a dialysis membrane
will be caused by the presence of hydrogen ions to
provide a color indication in the optical detection
range without diffusing therethrough, and which will
provide an indication of pH within the range of interest.
A discussion of such dye material will be found in U.S.
Patent 4,200,110 to John I. Peterson and Seth R.
Goldstein.

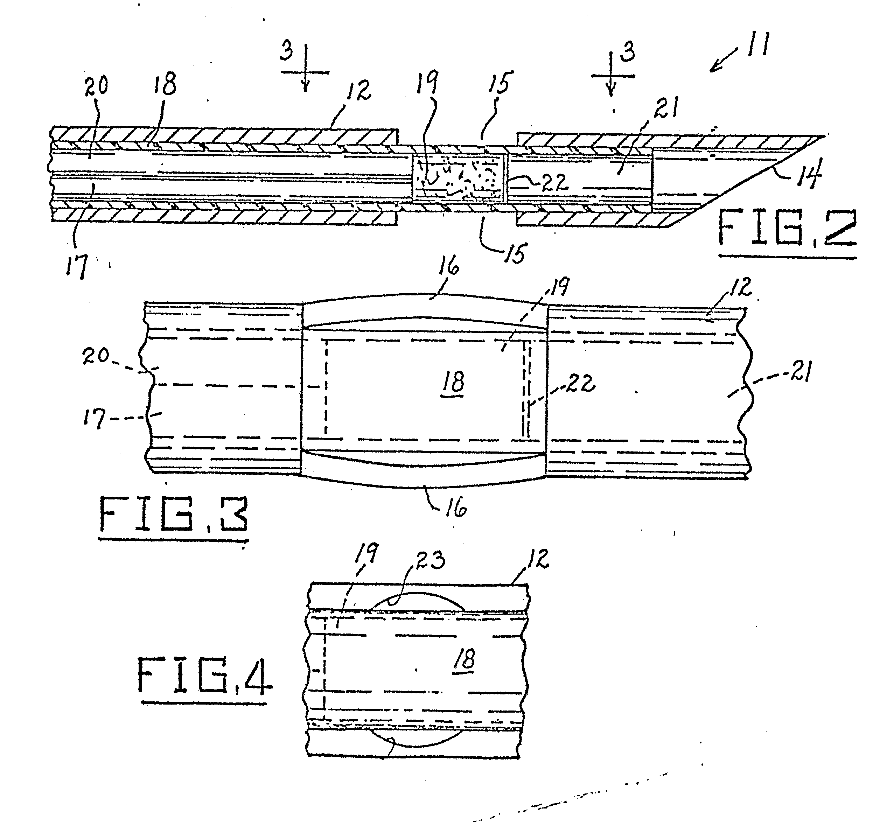

The length of the exposure slots 15, 15 is
sufficient to substantially span the length of the dye
column 19 with a small amount of overlap at each end,
as shown in Fig. 2. A reflector member 21, which may
comprise a 0.5 mm long glass rod with a vapor-
deposited metallic coating 22 is secured inside the
forward end portion of the dialysis tubing 18, as
shown in Fig. 2, forwardly contiguous with the forward
end of the dye column 19. The deposited-metal coating
22 serves as a mirror to enhance reflection and increase
the transmission of light through the dye path, thus
increasing the dye signal. The reflecting element
22 also eliminates optical artifacts, which in
previous probes often resulted from various optical
conditions at the end of the probe, and thus required
the use of a relatively long dye column.

The dialysis tube 18 and its contents are fixedly
secured inside the hollow needle 12 in a position such

0073558

as is shown in Fig. 2, with the dye column 19 completely contained within the exposure slots 15, 15. The outward spreading of the opposite residual wall portions 16, 16 allows hydrogen ions to gain access to the dialysis tubing 18 over the entire area in the region of the dye column 19. Alternatively, as shown in Fig. 4, the space in the needle portion 23, 23 created by the drilled hole allows hydrogen ions to gain access to the dialysis tubing 18 over the entire area of the dye column 19.

The arrangement above-described allows the use of a relatively short dye column 19, which shortens the required length of the probe and which provides greater localization of the measurement. Also, tissue damage is minimized, since the insertion and retraction of a large guide needle is not required.

The dye capsule and light path are protected from physical trauma, since they are located inside the needle 12 and said needle is easily insertable into tissue.

A suitable light source is coupled to the fiber 17 and a suitable light sensor is coupled to the remaining fiber 20, as shown in Fig. 2.

The determination of pH is desirable in a wide variety of biological studies, for example, for studies of blood oxygen content or in connection with studies of diseases where it is desirable to determine an oxygen-hemoglobin dissociation curve in vivo during exercise. In such studies the needle 12 is inserted in muscle or other tissue of interest. The probe operates on the principle of optically detecting and quantitatively measuring the change in color of the pH-sensitive dye in the column 19.

While a apecific embodiment of an improved fiber optic pH probe has been disclosed in the foregoing description, it will be understood that various modifications within the scope of the invention may occur to those skilled in the art.  Therefore it is intended that adaptations and modifications should and are intended to be comprehended within the meaning and range of equivalents of the disclosed embodiment.

-8-

## CLAIMS

1. A fiber optic pH probe suitable for insertion into tissue for physiological studies comprising a substantially rigid hollow needle with a sharp forward end, an ion-permeable membrane in the form of an ion-permeable tube mounted in said needle, said needle being provided with at least one aperture exposing said ion-permeable tube, pH sensitive color-changing dye-containing material mounted in said tube in the region of said aperture, means sealing the forward portion of the tube adjacent said dye-containing material, and a pair of optical fibers mounted in the tube rearwardly of and substantially contiguous to said dye-containing material for respectively delivering light from an external source to said dye-containing material and returning light from the dye-containing material to an external light sensor for measuring color change of the dye-containing material.

2. The pH probe of claim 1, and wherein said sealing means has a reflective end portion facing the dye-containing material for reflecting light passing through the dye-containing material.

3. The pH probe of claim 1, and wherein said sealing means comprises a rod-like member sealingly mounted in said tube and having a light-reflecting end facing the dye-containing material for reflecting light passing through the dye-containing material.

4. The pH probe of claim 3, and wherein said light-reflecting end comprises a vapor-deposited reflective inner metallic coating on the end of the rod-like member facing the dye-containing material.

5. The pH probe of claim 1, and wherein said needle is formed with a plurality of apertures exposing said ion-permeable tube in the region of the dye-containing material.

6. The pH probe of claim 1, and wherein said needle is formed with a pair of opposite apertures exposing said ion-permeable tube in the region of said dye-containing material.

7. The pH probe of claim 6, and wherein the residual wall portion of the needle between the apertures is spread outwardly to permit hydrogen ions to have access to the ion-permeable tube over the entire area thereof in the region of the dye-containing material.

8. The pH probe of claim 6, and wherein the residual wall portion of the needle between the apertures is thinned along the inside thereof to permit hydrogen ions to have access to the ion-permeable tube over the entire area thereof in the region of the dye-containing material.

9. The pH probe of claim 1, and wherein said needle is formed with a pair of opposed slots exposing opposite sides of said ion-permeable tube, and wherein the length of said slots substantially spans the length of said dye-containing material.

10. The pH probe of claim 9, and wherein said slots define substantially straight residual needle wall elements therebetween, and wherein said residual wall elements are spread outwardly to provide access of ions around the entire surface of the ion-permeable tube in the region of the dye-containing material.

11. The pH probe of claim 9, and wherein said slots define substantially straight residual needle wall elements therebetween, and wherein said residual wall elements are internally thinned to provide access of ions around the entire surface of the ion-permeable tube in the region of the dye-containing material.

12. The pH probe of claim 10, and wherein said sealing means comprises a rigid rod-like member secured in the tube forwardly adjacent to the dye-containing material and being provided with a metallic reflective layer facing the dye-containing material.

LIGHT SENSOR

LIGHT SOURCE

20

12

13

15

16

17

16

15

18

14

19

21

FIG.1

0073558

FIG.2

FIG.3

FIG.4